# EUROPEAN PATENT SPECIFICATION

(11) **EP 3 344 994 B1**
(45) Date of publication and mention of the grant of the patent: **13.11.2019**
(21) Application number: 16766739.3
(22) Date of filing: 01.09.2016
(51) Int. Cl.: G01N 33/497, A24F 47/00, A61B 5/08

(54) **METHOD FOR MONITORING USE OF A TOBACCO PRODUCT**
VERFAHREN ZUR ÜBERWACHUNG DER VERWENDUNG EINES TABAKPRODUKTES
PROCÉDÉ POUR SUIVRE L'UTILISATION D'UN PRODUIT DE TABAC

(30) Priority: 02.09.2015 US 201514843432
(43) Date of publication of application: 11.07.2018
(73) Proprietor: R. J. Reynolds Tobacco Company, Winston-Salem, NC 27101 (US)
(72) Inventor: DULL, Gary, Winston-Salem, North Carolina 27101 (US)
(74) Representative: Dehns
(86) International application number: PCT/US2016/049912
(87) International publication number: WO 2017/040789

(56) References cited:
- US-A1- 2010 137 733
- US-A1- 2014 378 790
- MELISSA HAGAN HUGHES ET AL: "Estimation of the Retention of Menthol in the Respiratory Tract of Menthol Cigarette Smokers: A Pilot Study", CONTRIBUTIONS TO TOBACCO RESEARCH, vol. 26, no. 1, 1 May 2014 (2014-05-01), pages 26-33, XP55314158, ISSN: 0005-819X, DOI: 10.2478/cttr-2014-0005
- ERIC T MOOLCHAN ET AL: "Heart rate and blood pressure responses to tobacco smoking among African-American adolescents", JOURNAL OF THE NATIONAL MEDICAL ASSOCIATION, vol. 96, no. 6, June 2004 (2004-06), pages 767-771, XP55314175, United States ISSN: 1943-4693

## Description

### FIELD OF THE INVENTION

A method such as is described in herein relates to monitoring use of a tobacco product by a subject. The method disclosed herein comprises providing the product to the subject and, at one or more times after providing the product, collecting a sample of exhaled breath from the subject and subjecting the collected sample to analysis to determine amount and/or concentration of a marker, wherein the amount and/or concentration of the marker is related to use of the product by the subject.

### BACKGROUND OF THE INVENTION

Popular smoking articles, such as cigarettes, have a substantially cylindrical rod shaped structure and include a charge, roll or column of smokable material such as shredded tobacco (e.g., in cut filler form) surrounded by a paper wrapper thereby forming a so-called "tobacco rod." Normally, a cigarette has a cylindrical filter element aligned in an end-to-end relationship with the tobacco rod. Typically, a filter element comprises plasticized cellulose acetate tow circumscribed by a paper material known as "plug wrap." Certain cigarettes incorporate a filter element having multiple segments, and one of those segments can comprise activated charcoal particles. Typically, the filter element is attached to one end of the tobacco rod using a circumscribing wrapping material known as "tipping paper." It also has become desirable to perforate the tipping material and plug wrap, in order to provide dilution of drawn mainstream smoke with ambient air. A cigarette is employed by a smoker by lighting one end thereof and burning the tobacco rod. The smoker then receives mainstream smoke into his/her mouth by drawing on the opposite end (e.g., the filter end) of the cigarette.

The tobacco used for cigarette manufacture is typically used in blended form. For example, certain popular tobacco blends, commonly referred to as "American blends," comprise mixtures of flue-cured tobacco, burley tobacco, and Oriental tobacco, and in many cases, certain processed tobaccos, such as reconstituted tobacco and processed tobacco stems. The precise amount of each type of tobacco within a tobacco blend used for the manufacture of a particular cigarette brand varies from brand to brand. However, for many tobacco blends, flue-cured tobacco makes up a relatively large proportion of the blend, while Oriental tobacco makes up a relatively small proportion of the blend. See, for example, Tobacco Encyclopedia, Voges (Ed.) p. 44-45 (1984), Browne, The Design of Cigarettes, 3rd Ed., p. 43 (1990) and Tobacco Production, Chemistry and Technology, Davis et al. (Eds.) p. 346 (1999).

Through the years, various treatment methods and additives have been proposed for altering the overall character or nature of tobacco materials utilized in tobacco products. For example, additives or treatment processes have been utilized in order to alter the chemistry or sensory properties of the tobacco material, or in the case of smokable tobacco materials, to alter the chemistry or sensory properties of mainstream smoke generated by smoking articles including the tobacco material. The sensory attributes of cigarette smoke can be enhanced by incorporating flavoring materials into various components of a cigarette. Exemplary flavoring additives include menthol and products of Maillard reactions, such as pyrazines, aminosugars, and Amadori compounds. See also, Leffingwell et al., Tobacco Flavoring for Smoking Products, R.J. Reynolds Tobacco Company (1972). In some cases, treatment processes involving the use of heat can impart to the processed tobacco a desired color or visual character, desired sensory properties, or a desired physical nature or texture. Various processes for preparing flavorful and aromatic compositions for use in tobacco compositions are set forth in US Pat. Nos. 3,424,171 to Rooker; 3,476,118 to Luttich; 4,150,677 to Osborne, Jr. et al.; 4,986,286 to Roberts et al.; 5,074,319 to White et al.; 5,099,862 to White et al.; 5,235,992 to Sensabaugh, Jr.; 5,301,694 to Raymond et al.; 6,298,858 to Coleman, III et al.; 6,325,860 to Coleman, III et al.; 6,428,624 to Coleman, III et al.; 6,440,223 to Dube et al.; 6,499,489 to Coleman, III; 6,591,841 to White et al.; and 6,695,924 to Dube et al.; and US Pat. Appl. Publication Nos. 2004/0173228 to Coleman, III; 2010/0037903 to Coleman, III et al.; and 2013/0014771 to Coleman, III et al. Additionally, examples of representative components that can be employed as so-called natural tar diluents in tobacco products are set in PCT WO 07/012980 to Lipowicz.

Tobacco also may be enjoyed in a so-called "smokeless" form. Particularly popular smokeless tobacco products are employed by inserting some form of processed tobacco or tobacco-containing formulation into the mouth of the user. Various types of smokeless tobacco products are set forth in US Pat. Nos. 1,376,586 to Schwartz; 3,696,917 to Levi; 4,513,756 to Pittman et al.; 4,528,993 to Sensabaugh, Jr. et al.; 4,624,269 to Story et al.; 4,987,907 to Townsend; 5,092,352 to Sprinkle, III et al.; 5,387,416 to White et al.; and 8,336,557 to Kumar et al.; US Pat. Appl. Pub. Nos. 2005/0244521 to Strickland et al. and 2008/0196730 to Engstrom et al.; PCT WO 04/095959 to Arnarp et al.; PCT WO 05/063060 to Atchley et al.; PCT WO 05/016036 to Bjorkholm; and PCT WO 05/041699 to Quinter et al.. See, for example, the types of smokeless tobacco formulations, ingredients, and processing methodologies set forth in US Pat. Nos. 6,953,040 to Atchley et al. and 7,032,601 to Atchley et al.

One type of smokeless tobacco product is referred to as "snuff." Representative types of moist snuff products, commonly referred to as "snus," have been manufactured in Europe, particularly in Sweden, by or through companies such as Swedish Match AB, Fiedler & Lundgren AB, Gustavus AB, Skandinavisk Tobakskompagni A/S, and Rocker Production AB. Snus products available in the U.S.A. have been marketed under the tradenames Camel Snus Frost, Camel Snus Original and Camel Snus Spice by R. J. Reynolds Tobacco Company. See also, for example, Bryzgalov et al., 1N1800 Life Cycle Assessment, Comparative Life Cycle Assessment of General Loose and Portion Snus (2005). In addition, certain quality standards associated with snus manufacture have been assembled as a so-called GothiaTek standard. Representative smokeless tobacco products also have been marketed under the tradenames Oliver Twist by House of Oliver Twist A/S; Copenhagen, Skoal, SkoalDry, Rooster, Red Seal, Husky, and Revel by U.S. Smokeless Tobacco Co.; "taboka" by Philip Morris USA; Levi Garrett, Peachy, Taylor's Pride, Kodiak, Hawken Wintergreen, Grizzly, Dental, Kentucky King, and Mammoth Cave by Conwood Company, LLC; and Camel Orbs, Camel Sticks, and Camel Strips by R. J. Reynolds Tobacco Company.

The sensory attributes of smokeless tobacco can also be enhanced by incorporation of certain flavoring materials. See, for example, US Pat. Nos. 6,668,839 to Williams; 6,834,654 to Williams; 7,032,601 to Atchley et al.; 7,694,686 to Atchley et al.; 7,861,728 to Holton, Jr. et al.; 7,819,124 to Strickland et al.; 7,810,507 to Dube et al.; and 8,168,855 to Nielsen et al; US Pat. Appl. Pub. Nos. 2004/0020503 to Williams, 2006/0191548 to Strickland et al.; 2007/0062549 to Holton, Jr. et al.; 2008/0029116 to Robinson et al.; 2008/0029117 to Mua et al.; and 2008/0173317 to Robinson et al.

A tobacco product may be a so-called electronic cigarette. The liquid in an electronic cigarette may comprise, for example, propylene glycol or glycerin, and may further comprise one or more flavorants, and may further comprise one or more substances that may impart a favorable user experience. A so-called electronic cigarette is an example of an alternative smoking device. Many such alternative smoking devices have been proposed through the years as improvements upon, or alternatives to, smoking products that require combusting tobacco for use. Many of those devices purportedly have been designed to provide the sensations associated with cigarette, cigar, or pipe smoking, but without delivering considerable quantities of incomplete combustion and pyrolysis products that result from the burning of tobacco. To this end, there have been proposed numerous smoking products, flavor generators, and medicinal inhalers that utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. No. 7,726,320 to Robinson et al., U.S. Pat. Pub. No. 2013/0255702 to Griffith Jr. et al., U.S. Pat. Pub. Nos. 2014/0000638 to Sebastian et al., 2014/0060554 to Collett et al., 2014/0060555 to Chang et al., 2014/0096781 to Sears et al., 2014/0096782 to Ampolini et al., and 2015/0059780 to Davis et al.

Because tobacco products have long been consumed, yet have there been few if any ways reliably to monitor consumption of such products, there is a long-felt need for a method for monitoring use of tobacco products.

US 2014/378790 describes methods, systems and devices for providing risk evaluation and mitigation strategies for use with tobacco products. In particular, it describes a method of monitoring tobacco use by a subject by taking a sample of the subject's breath after the subject's use of a tobacco product and measuring levels of a marker associated with tobacco use (e.g. nicotine levels, carbon monoxide) in the sample.

Hughes et al., 2014 (Contributions to Tobacco Research, vol. 26(1), pp. 26-33) describe a study designed to determine the deposition and retention efficiency of methanol in cigarette smoke in the respiratory tract when smoking mentholated cigarettes in which analytes in exhaled cigarette smoke were analysed.

Moolchan et al., 2004 (Journal of the National Medical Association, vol. 96(6) pp. 767-771) describe a study comparing acute physiological responses to smoking menthol cigarettes in different ethnic groups by measuring blood pressure, heart rate and exhaled carbon monoxide concentrations before and after smoking.

### SUMMARY OF EMBODIMENTS

The invention provides a method for monitoring use of a tobacco product by a subject, comprising providing the product to the subject and, at one or more times after providing the product, collecting a sample of exhaled breath from the subject and subjecting the collected sample to analysis to determine amount and/or concentration of a marker, wherein the amount and/or concentration of the marker is related to use of the product by the subject, wherein:
the tobacco product comprises a flavorant;
the marker is derived from the flavorant; and
the marker comprises 2-propanol, 2-butanol, 2-pentanol, 3-pentanol, 3-methyl-2-butanol, 3-hexanol, 2-hexanol, 3-methyl-2-pentanol, 4-methyl-2-pentanol, 2,4-dimethyl-3-pentanol, 3-methyl-3-hexanol, 2,6-dimethyl-4-heptanol, 2-heptanol, 3-heptanol, 4-heptanol, 5-methyl-3-heptanol, 6-methyl-3-heptanol, cyclopentanol, cyclohexanol, 4-isopropylcyclohexanol, and/or trimethylcyclohexanol.
Products to which a method such as is described in various embodiments herein can be applied can vary, and include without limitation any tobacco product that can be consumed in any form, including, for example, a smoking article, a smokeless tobacco product, a dissolvable (such as gums, lozenges, snus, spray, orbs and sticks), or a so-called electronic cigarette.

When used in connection with a process such as is described in various embodiments herein, the term "one or more plants of genus *Nicotiana"* denotes any one or more plants of the genus *Nicotiana* of family *Solanaceae,* including, for example, any one or more of the following: *N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata,* and *N. x sanderae, N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. rustica, N. simulans, N. stocktonii, N. suaveolens, N. tabacum, N. umbratica, N. velutina,* and *N. wigandioides, N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis* subsp. *Hersperis, N. pauciflora, N. petunioides, N. quadrivalvis, N. repanda, N. rotundifolia, N. solanifolia, N. spegazzinii.*

The use of *Nicotiana-*derived (e.g., tobacco-derived) materials monitored by a method such as is described in various embodiments herein enables monitoring of use of smoking articles or smokeless tobacco compositions that are derived substantially or even entirely from *Nicotiana* materials. For example, a tobacco composition can incorporate tobacco or tobacco-derived material of some form, including isolated components from *Nicotiana* species, such that at least about 80 weight percent, more typically at least about 90 weight percent, or even at least about 95 weight percent (on a dry weight basis), of that tobacco composition consists of tobacco-derived material.

### DETAILED DESCRIPTION

A method such as is described in various embodiments herein now will be described more fully hereinafter. As used in this specification and the claims, the singular forms "a," "an," and "the" include plural referents unless the context clearly dictates otherwise. Any reference to "dry weight percent" or "dry weight basis" refers to weight on the basis of dry ingredients (i.e., all ingredients except water). When used in this specification and the claims as an adverb rather than a preposition, "about" means "approximately" and comprises the stated value and every value within 10% of that value; in other words, "about 100%" includes 90% and 110% and every value in between.

The selection of the plant from a *Nicotiana* species can vary; and in particular, the types of tobacco or tobaccos may vary. Tobaccos that can be employed include flue-cured or Virginia (e.g., K326), burley, sun-cured (e.g., Indian Kurnool and Oriental tobaccos, including Katerini, Prelip, Komotini, Xanthi and Yambol tobaccos), Maryland, dark, dark-fired, dark air cured (e.g., Passanda, Cubano, Jatin and Bezuki tobaccos), light air cured (e.g., North Wisconsin and Galpao tobaccos), Indian air cured, Red Russian and Rustica tobaccos, as well as various other rare or specialty tobaccos. Descriptions of various types of tobaccos, growing practices and harvesting practices are set forth in Tobacco Production, Chemistry and Technology, Davis et al. (Eds.) (1999). Various representative types of plants from the Nicotiana species are set forth in Goodspeed, The Genus Nicotiana (Chronica Botanica, 1954); US Pat. Nos. 4,660,577 to Sensabaugh, Jr. et al.; 5,387,416 to White et al.; 7,025,066 to Lawson et al.; 7,798,153 to Lawrence, Jr.; and 8,186,360 to Marshall et al. Of particular interest are *N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata,* and *N. x sanderae.* Also of interest are *N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. rustica, N. simulans, N. stocktonii, N. suaveolens, N. tabacum, N. umbratica, N. velutina,* and *N. wigandioides.* Other plants from the *Nicotiana* species include *N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis* subsp. *Hersperis, N. pauciflora, N. petunioides, N. quadrivalvis, N. repanda, N. rotundifolia, N. solanifolia* and *N. spegazzinii.*

*Nicotiana* species can be derived using genetic-modification or crossbreeding techniques (e.g., tobacco plants can be genetically engineered or crossbred to increase or decrease production of certain components or to otherwise change certain characteristics or attributes). See, for example, the types of genetic modifications of plants set forth in US Pat. Nos. 5,539,093 to Fitzmaurice et al.; 5,668,295 to Wahab et al.; 5,705,624 to Fitzmaurice et al.; 5,844,119 to Weigl; 6,730,832 to Dominguez et al.; 7,173,170 to Liu et al.; 7,208,659 to Colliver et al.; and 7,230,160 to Benning et al.; US Patent Appl. Pub. No. 2006/0236434 to Conkling et al.; and PCT WO 08/103935 to Nielsen et al.

For the preparation of smokeless and smokable tobacco products, it is typical for harvested plants of a *Nicotiana* species to be subjected to a curing process. Descriptions of various types of curing processes for various types of tobaccos are set forth in Tobacco Production, Chemistry and Technology, Davis et al. (Eds.) (1999). Exemplary techniques and conditions for curing flue-cured tobacco are set forth in Nestor et al., Beitrage Tabakforsch. Int., 20, 467-475 (2003) and U.S. Pat. No. 6,895,974 to Peele. See, also, for example, US Pat. No. 7,650,892 to Groves et al. Representative techniques and conditions for air curing tobacco are set forth in Roton et al., Beitrage Tabakforsch. Int., 21, 305-320 (2005) and Staaf et al., Beitrage Tabakforsch. Int., 21, 321-330 (2005). Certain types of tobaccos can be subjected to alternative types of curing processes, such as fire curing or sun curing. Preferably, harvested tobaccos that are cured are then aged.

At least a portion of the plant of a *Nicotiana* species (e.g., at least a portion of the tobacco portion) can be employed in an immature form. That is, the plant, or at least one portion of that plant, can be harvested before reaching a stage normally regarded as ripe or mature. As such, for example, tobacco can be harvested when the tobacco plant is at the point of a sprout, is commencing leaf formation, is commencing seeding, is commencing flowering, or the like.

At least a portion of the plant of a *Nicotiana* species (e.g., at least a portion of the tobacco portion) can be employed in a mature form. That is, the plant, or at least one portion of that plant, can be harvested when that plant (or plant portion) reaches a point that is traditionally viewed as being ripe, over-ripe or mature. As such, for example, through the use of tobacco harvesting techniques conventionally employed by farmers, Oriental tobacco plants can be harvested, burley tobacco plants can be harvested, or Virginia tobacco leaves can be harvested or primed by stalk position. After harvest, a plant of a *Nicotiana* species, or portion thereof, can be used in a green form (e.g., tobacco can be used without being subjected to any curing process). For example, tobacco in green form can be frozen, freeze-dried, subjected to irradiation, yellowed, dried, cooked (e.g., roasted, fried or boiled), or otherwise subjected to storage or treatment for later use. Such tobacco also can be subjected to aging conditions.

In accordance with a method such as is described in various embodiments herein, a tobacco product may incorporate tobacco that is combined with some form of biomass or one or more anatomical parts obtained from, or derived from, a plant of at least one *Nicotiana* species. That is, a portion of a tobacco product according to a method such as is described in various embodiments herein can be composed of some form of biomass or one or more anatomical parts of a *Nicotiana* species, such as parts or pieces of biomass or one or more anatomical parts, or processed materials incorporating processed biomass or one or more anatomical parts or components thereof. At least a portion of the tobacco product can be composed of components of biomass or one or more anatomical parts, such as ingredients removed from biomass or one or more anatomical parts (e.g., by extraction, distillation, or other types of processing techniques). At least a portion of the tobacco product can be composed of components derived from biomass or one or more anatomical parts, such as components collected after subjecting biomass or one or more anatomical parts to chemical reaction or after subjecting components collected from biomass or one or more anatomical parts to chemical reaction (e.g., acid/base reaction conditions or enzymatic treatment).

A *Nicotiana* species can be selected for the type of biomass or anatomical part that it produces. For example, plants can be selected on the basis that those plants produce relatively abundant biomass or seed, produce biomass or seed that incorporate relatively high levels of specific desired components, and the like.

A *Nicotiana* species of plant can be grown under agronomic conditions so as to promote development of biomass or one or more anatomical parts. Tobacco plants can be grown in greenhouses, growth chambers, or outdoors in fields, or grown hydroponically.

According to a method such as is described in various embodiments herein, biomass or one or more anatomical parts are harvested from a *Nicotiana* species of plant. The manner by which biomass or one or more anatomical parts are harvested can vary. Typically, essentially all the biomass or anatomical parts can be harvested, and employed as such.

Time of harvest during the life cycle of the plant can vary. For example, biomass or one or more anatomical parts can be harvested when immature. Alternatively, biomass or one or more anatomical parts can be harvested after the point that the plant has reached maturity.

Post-harvest processing of biomass or one or more anatomical parts can vary. After harvest, the biomass or one or more anatomical parts, or portion thereof, can be used in the harvested form (e.g., the biomass or one or more anatomical parts, or portion thereof, can be used without being subjected to any curing and/or aging process steps). For example, biomass or one or more anatomical parts can be used without being subjected to significant storage, handling or processing conditions. In certain situations, it is preferable that fresh biomass or one or more anatomical parts be used virtually immediately after harvest. Alternatively, for example, biomass or one or more anatomical parts can be refrigerated or frozen for later use, freeze dried, subjected to irradiation, yellowed, dried, cured (e.g., using air drying techniques or techniques that employ application of heat), heated or cooked (e.g., roasted, fried or boiled), or otherwise subjected to storage or treatment for later use.

Harvested biomass can be physically processed. Biomass or one or more anatomical parts, or one or more parts thereof, can be further subdivided into parts or pieces (e.g., biomass can be comminuted, pulverized, milled or ground into pieces or parts that can be characterized as granules, particulates or fine powders, or, e.g., petals can be removed from remaining portion of a flower). Biomass or one or more anatomical parts, or one or more parts thereof, can be subjected to external forces or pressure (e.g., by being pressed or subjected to roll treatment). When carrying out such processing conditions, biomass or one or more anatomical parts can have a moisture content that approximates its natural moisture content (e.g., its moisture content immediately upon harvest), a moisture content achieved by adding moisture to the biomass, or a moisture content that results from the drying of the biomass. For example, powdered, pulverized, ground or milled pieces of biomass or one or more anatomical parts can have moisture contents of less than about 25 weight percent, often less than about 20 weight percent, and frequently less than about 15 weight percent. Parts or pieces of biomass or one or more anatomical parts can be used as components of tobacco products without further processing, or alternatively the particulate biomass or anatomical part material can be processed further prior to incorporation into a tobacco product.

Harvested biomass or one or more anatomical parts or components thereof can be subjected to other types of processing conditions. For example, components of biomass or one or more anatomical parts can be separated from one another, or otherwise fractionated into chemical classes or mixtures of individual compounds. As used herein, an "isolated biomass component," "isolated component of one or more anatomical parts," "biomass isolate," "isolate of one or more anatomical parts," or "isolate" when used as a noun is a compound or complex mixture of compounds separated from biomass or one or more anatomical parts of a plant of a *Nicotiana* species. The isolated biomass component or isolated component of one or more anatomical parts can be a single compound, a homologous mixture of similar compounds (e.g., isomers of a flavorful or aromatic compound), or a heterologous mixture of dissimilar compounds (e.g., a complex mixture of various compounds of different types, preferably having desirable sensory attributes).

Typical separation processes can include one or more process steps such as solvent extraction (e.g., using polar solvents, non-polar organic solvents, or supercritical fluids), chromatography, distillation, filtration, cold pressing or other pressure-based techniques, recrystallization, and/or solvent-solvent partitioning. Exemplary extraction and separation solvents or carriers include water, alcohols (e.g., methanol or ethanol), hydrocarbons (e.g., heptane and hexane), diethyl ether, methylene chloride and supercritical carbon dioxide. Exemplary techniques useful for extracting components from *Nicotiana* species are described in US Pat. Nos. 4,144,895 to Fiore; 4,150,677 to Osborne, Jr. et al.; 4,267,847 to Reid; 4,289,147 to Wildman et al.; 4,351,346 to Brummer et al.; 4,359,059 to Brummer et al.; 4,506,682 to Muller; 4,589,428 to Keritsis; 4,605,016 to Soga et al.; 4,716,911 to Poulose et al.; 4,727,889 to Niven, Jr. et al.; 4,887,618 to Bernasek et al.; 4,941,484 to Clapp et al.; 4,967,771 to Fagg et al.; 4,986,286 to Roberts et al.; 5,005,593 to Fagg et al.; 5,018,540 to Grubbs et al.; 5,060,669 to White et al.; 5,065,775 to Fagg; 5,074,319 to White et al.; 5,099,862 to White et al.; 5,121,757 to White et al.; 5,131,414 to Fagg; 5,131,415 to Munoz et al.; 5,148,819 to Fagg; 5,197,494 to Kramer; 5,230,354 to Smith et al.; 5,234,008 to Fagg; 5,243,999 to Smith; 5,301,694 to Raymond et al.; 5,318,050 to Gonzalez-Parra et al.; 5,343,879 to Teague; 5,360,022 to Newton; 5,435,325 to Clapp et al.; 5,445,169 to Brinkley et al.; 6,131,584 to Lauterbach; 6,298,859 to Kierulff et al.; 6,772,767 to Mua et al.; and 7,337,782 to Thompson. See also, the types of separation techniques set forth in Brandt et al., LC-GC Europe, p. 2-5 (March, 2002) and Wellings, A Practical Handbook of Preparative HPLC (2006). In addition, the biomass or components thereof can be subjected to the types of treatments set forth in Ishikawa et al., Chem. Pharm. Bull., 50, 501-507 (2002); Tienpont et al., Anal. Bioanal. Chem., 373, 46-55 (2002); Ochiai, Gerstel Solutions Worldwide, 6, 17-19 (2006); Coleman, III, et al., J. Sci. Food and Agric., 84, 1223-1228 (2004); Coleman, III et al., J. Sci. Food and Agric., 85, 2645-2654 (2005); Pawliszyn, ed., Applications of Solid Phase Microextraction, RSC Chromatography Monographs, (Royal Society of Chemistry, UK) (1999); Sahraoui et al., J. Chrom., 1210, 229-233 (2008); and US Pat. No. 5,301,694 to Raymond et al. See also, for example, the types of processing techniques set forth in Frega et al., JAOCS, 68, 29-33 (1991); Patel et al., Tob. Res., 24, 44-49 (1998); Giannelos et al., Ind. Crops Prod., 16, 1-9 (2002); Mukhtar et al., Chinese J. Chem., 25, 705-708 (2007); and Stanisavljevic et al., Eur. J. Lipid Sci. Technol., 111, 513-518 (2009).

A portion of a harvested tobacco plant can be employed in any of a variety of forms. Harvested biomass or one or more anatomical parts can be employed as a component of processed tobaccos. In one regard, harvested biomass or one or more anatomical parts can be employed within a casing formulation for application to tobacco strip (e.g., using the types of manners and methods set forth in U.S. Pat. No. 4,819,668 to Shelar) or within a top dressing formulation. Alternatively, harvested biomass or one or more anatomical parts can be employed as an ingredient of a reconstituted tobacco material (e.g., using the types of tobacco reconstitution processes generally set forth in U.S. Pat. No. 5,143,097 to Sohn; U.S. Pat. No. 5,159,942 to Brinkley et al.; U.S. Pat. No. 5,598,868 to Jakob; U.S. Pat. No. 5,715,844 to Young; U.S. Pat. No. 5,724,998 to Gellatly; and U.S. Pat. No. 6,216,706 to Kumar). Harvested biomass or one or more anatomical parts also can be incorporated into a cigarette filter (e.g., in the filter plug, plug wrap, or tipping paper) or incorporated into cigarette wrapping paper, preferably on the inside surface, during the cigarette manufacturing process.

Harvested biomass or one or more anatomical parts can be incorporated into smoking articles. Representative tobacco blends, non-tobacco components, and representative cigarettes manufactured therefrom, are set forth in U.S. Pat. No. 4,836,224 to Lawson et al.; U.S. Pat. No. 4,924,888 to Perfetti et al.; U.S. Pat. No. 5,056,537 to Brown et al.; U.S. Pat. No. 5,220,930 to Gentry; and U.S. Pat. No. 5,360,023 to Blakley et al.; US Pat. Application 2002/0000235 to Shafer et al.; and PCT WO 02/37990. Those tobacco materials also can be employed for the manufacture of those types of cigarettes that are described in U.S. Pat. No. 4,793,365 to Sensabaugh; U.S. Pat. No. 4,917,128 to Clearman et al.; U.S. Pat. No. 4,947,874 to Brooks et al.; U.S. Pat. No. 4,961,438 to Korte; U.S. Pat. No. 4,920,990 to Lawrence et al.; U.S. Pat. No. 5,033,483 to Clearman et al.; U.S. Pat. No. 5,074,321 to Gentry et al.; U.S. Pat. No. 5,105,835 to Drewett et al.; U.S. Pat. No. 5,178,167 to Riggs et al.; U.S. Pat. No. 5,183,062 to Clearman et al.; U.S. Pat. No. 5,211,684 to Shannon et al.; U.S. Pat. No. 5,247,949 to Deevi et al.; U.S. Pat. No. 5,551,451 to Riggs et al.; U.S. Pat. No. 5,285,798 to Banerjee et al.; U.S. Pat. No. 5,593,792 to Farrier et al.; U.S. Pat. No. 5,595,577 to Bensalem et al.; U.S. Pat. No. 5,816,263 to Counts et al.; U.S. Pat. No. 5,819,751 to Barnes et al.; U.S. Pat. No. 6,095,153 to Beven et al.; U.S. Pat. No. 6,311,694 to Nichols et al.; and U.S. Pat. No. 6,367,481 to Nichols, et al.; US Pat. Appl. Pub. No. 2008/0092912 to Robinson et al.; and PCT WO 97/48294 and PCT WO 98/16125. See, also, those types of commercially marketed cigarettes described Chemical and Biological Studies on New Cigarette Prototypes that Heat Instead of Burn Tobacco, R. J. Reynolds Tobacco Company Monograph (1988) and Inhalation Toxicology, 12:5, p. 1-58 (2000).

Harvested biomass or one or more anatomical parts can be incorporated into smokeless tobacco products, such as loose moist snuff, loose dry snuff, chewing tobacco, pelletized tobacco pieces (e.g., having the shapes of pills, tablets, spheres, coins, beads, obloids or beans), extruded or formed tobacco strips, pieces, rods, cylinders or sticks, finely divided ground powders, finely divided or milled agglomerates of powdered pieces and components, flake-like pieces, molded processed tobacco pieces, pieces of tobacco-containing gum, rolls of tape-like films, readily water-dissolvable or water-dispersible films or strips (e.g., US Pat. App. Pub. No. 2006/0198873 to Chan et al.), or capsule-like materials possessing an outer shell (e.g., a pliable or hard outer shell that can be clear, colorless, translucent or highly colored in nature) and an inner region possessing tobacco or tobacco flavor (e.g., a Newtonian fluid or a thixotropic fluid incorporating tobacco of some form). Various types of smokeless tobacco products are set forth in U.S. Pat. No. 1,376,586 to Schwartz; U.S. Pat. No. 3,696,917 to Levi; U.S. Pat. No. 4,513,756 to Pittman et al.; U.S. Pat. No. 4,528,993 to Sensabaugh, Jr. et al.; U.S. Pat. No. 4,624,269 to Story et al.; U.S. Pat. No. 4,987,907 to Townsend; U.S. Pat. No. 5,092,352 to Sprinkle, III et al.; and U.S. Pat. No. 5,387,416 to White et al.; US Pat. App. Pub. Nos. U.S. Pat. No. 2005/0244521 to Strickland et al. and U.S. Pat. No. 2008/0196730 to Engstrom et al.; PCT WO 04/095959 to Arnarp et al.; PCT WO 05/063060 to Atchley et al.; PCT WO 05/016036 to Bjorkholm; and PCT WO 05/041699 to Quinter et al. See also, the types of smokeless tobacco formulations, ingredients, and processing methodologies set forth in U.S. Pat. No. 6,953,040 to Atchley et al. and U.S. Pat. No. 7,032,601 to Atchley et al.; US Pat. Appl. Pub. Nos. 2002/0162562 to Williams; 2002/0162563 to Williams; 2003/0070687 to Atchley et al.; 2004/0020503 to Williams, 2005/0178398 to Breslin et al.; 2006/0191548 to Strickland et al.; 2007/0062549 to Holton, Jr. et al.; 2007/0186941 to Holton, Jr. et al.; 2007/0186942 to Strickland et al.; 2008/0029110 to Dube et al.; 2008/0029116 to Robinson et al.; 2008/0029117 to Mua et al.; 2008/0173317 to Robinson et al.; and 2008/0209586 to Nielsen et al.

Harvested biomass or one or more anatomical parts can be incorporated into an electronic smoking article. An electronic smoking article may encompass a variety of combinations of components useful in forming an electronic aerosol delivery device. Reference is made, for example, to the following: a reservoir and heater system for controllable delivery of multiple aerosolizable materials disclosed in US Pat. Pub. No. 2014/0000638 to Sebastian et al.; microheaters as disclosed in US Pat. Pub. No. 2014/0060554 to Collett et al.; carbon-based cartridges and components thereof, as disclosed US Pat. Pub. No. 2013/0255702 to Griffith, Jr. et al.; single-use cartridges as disclosed in US Pat. Pub. No. 2014/0060555 to Chang et al.; aerosol precursor transport elements, such as disclosed in US Pat. Pub. No. 2014/0209105 to Sears et al.; charging components, such as an adaptor disclosed in US Pat. Pub. No. 2014/0261495 to Novak, III et al.; vibration components, such as disclosed in US Pat. Pub. No. 2015/0020825 to Galloway et al.; and batteries, such as disclosed in U.S. Pat. Pub. No. 2010/0028766 to Peckerar et al.

Representative types of aerosol precursor components and formulations also are set forth and characterized in U.S. Pat. No. 7,217,320 to Robinson et al. and U.S. Pat. Pub. Nos. 2013/0008457 to Zheng et al.; 2013/0213417 to Chong et al.; 2014/0060554 to Collett et al.; 2015/0020823 to Lipowicz et al.; and 2015/0020830 to Koller, as well as WO 2014/182736 to Bowen et al. Other aerosol precursors that may be employed include the aerosol precursors that have been incorporated in the VUSE® product by R. J. Reynolds Vapor Company, the BLU™ product by Lorillard Technologies, the MISTIC MENTHOL product by Mistic Ecigs, and the VYPE product by CN Creative Ltd. Also desirable are the so-called "smoke juices" for electronic cigarettes that have been available from Johnson Creek Enterprises LLC. Additionally, a tobacco product may be, or may comprise, a medicinal or nicotine inhaler. A nicotine inhaler may be as set forth and characterized in US Pat. Pub. No. 2015/0208728, 2015/0201675, 2015/0196058, 2015/0157055, 2015/0136153, 2015/0128966, 2015/0128965 and/or 2015/0114408, each of which is to Lord. A tobacco product, such as a nicotine inhaler, may be, or may comprise, a Voke® nicotine inhaler such as is produced for and/or licensed to Nicoventures Ltd. London, UK.

An amount of harvested biomass or one or more anatomical parts added to a tobacco composition, or otherwise incorporated within a tobacco composition or tobacco product, can depend on the desired function of that harvested biomass or one or more anatomical parts, the chemical makeup of that component, and the type of tobacco composition to which the harvested biomass or one or more anatomical parts are added. When the harvested biomass or one or more anatomical parts comprise a flower or flower isolate, for example, the amount added to a tobacco composition can vary, but will typically not exceed about 5 weight percent based on the total dry weight of the tobacco composition to which the flower or flower isolate or seed or seed isolate is added. When the flower is employed within a smoking article, the amount of flower will typically be at least about 5 ppm, generally at least about 10 ppm, and often at least about 100 ppm, based on the total dry weight of the tobacco material within the smoking article; but will typically be less than about 5 percent, generally less than 2 percent, and often less than about 1 percent, based on the total dry weight of the tobacco material within the smoking article. When the flower is employed within a smokeless tobacco product, the amount of flower will typically be less at least about 5 ppm, generally at least about 10 ppm, and often at least about 100 ppm, based on the total dry weight of the tobacco material within the smokeless tobacco product; but will typically be less than about 5 percent, generally less than 2 percent, and often less than about 1 percent, based on the total dry weight of the tobacco material within the smokeless tobacco product.

Aspects of a method such as is described in various embodiments herein are further illustrated by the following examples, which are set forth to illustrate certain aspects of a method such as is described in various embodiments herein and are not to be construed as limiting thereof.

In an example, a method such as is described in various embodiments herein comprises providing a tobacco product comprising one or more flavorants listed in the FEMA GRAS™ Flavoring Substance List published by the Flavor and Extract Manufacturers Association. In accordance with the invention, a subject's consumption of a tobacco product comprising such a flavorant is monitored by detection of a marker derived from the flavorant in exhaled breath of the subject, wherein the marker is selected from 2-propanol, 2-butanol, 2-pentanol, 3-pentanol, 3-methyl-2-butanol, 3-hexanol, 2-hexanol, 3-methyl-2-pentanol, 4-methyl-2-pentanol, 2,4-dimethyl-3-pentanol, 3-methyl-3-hexanol, 2,6-dimethyl-4-heptanol, 2-heptanol, 3-heptanol, 4-heptanol, 5-methyl-3-heptanol, 6-methyl-3-heptanol, cyclopentanol, cyclohexanol, 4-isopropylcyclohexanol, and/or trimethylcyclohexanol.

The method described in various embodiments herein comprises collecting one or more samples of exhaled breath from a subject. Such one or more samples are subjected to analysis to determine one or more baseline marker values.

The method described in various embodiments herein comprises providing a tobacco product to the subject after one or more samples of exhaled breath have been collected from the subject for the determination of one or more baseline marker values.

The method described in various embodiments herein comprises collecting one or more samples of exhaled breath from a subject after a tobacco product has been provided to the subject. Such one or more samples are subjected to analysis to determine one or more experimental marker values. By "experimental" is meant that the value is determined once a study has begun, or subsequent to conduct of the study.

In an example, a SMART™ analytical system provided by Xhale, Inc., of Gainesville, Florida, is employed for conduct of analyses, see e.g. US Pat. Appl. Pub. No. 2014/0341983 to Dennis and Melker.

In an example, concentration of a marker in a sample of exhaled breath is directly related to use of a tobacco product by a subject.

In an example, mass of a marker in a sample of exhaled breath is directly related to use of a tobacco product by a subject.

Many modifications and other embodiments of a method such as is described in various embodiments herein will come to mind to one skilled in the art to which this disclosed method pertains having the benefit of the teachings presented in the foregoing description.

In an example, a SMART™ analytical system provided by Xhale, Inc., of Gainesville, Florida, is employed for conduct of analyses.

In an example, concentration of a marker and/or a biomarker in a sample of exhaled breath is directly related to use of a tobacco product by a subject.

In an example, mass of a marker and/or a biomarker in a sample of exhaled breath is directly related to use of a tobacco product by a subject.

Many modifications and other embodiments of a method such as is described in various embodiments herein will come to mind to one skilled in the art to which this disclosed method pertains having the benefit of the teachings presented in the foregoing description. Therefore, it is to be understood that a method such as is described in various embodiments herein is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A method for monitoring use of a tobacco product by a subject, the method comprising providing the tobacco product to the subject and, at one or more times after providing the tobacco product, collecting a sample of exhaled breath from the subject and subjecting the collected sample to analysis to determine amount and/or concentration of a marker, wherein the amount and/or concentration of the marker is related to use of the tobacco product by the subject, thereby monitoring the use of the tobacco product by the subject, **characterized in that**:
the tobacco product comprises a flavorant;
the marker is derived from the flavorant; and
the marker comprises 2-propanol, 2-butanol, 2-pentanol, 3-pentanol, 3-methyl-2-butanol, 3-hexanol, 2-hexanol, 3-methyl-2-pentanol, 4-methyl-2-pentanol, 2,4-dimethyl-3-pentanol, 3-methyl-3-hexanol, 2,6-dimethyl-4-heptanol, 2-heptanol, 3-heptanol, 4-heptanol, 5-methyl-3-heptanol, 6-methyl-3-heptanol, cyclopentanol, cyclohexanol, 4-isopropylcyclohexanol, and/or trimethylcyclohexanol.

2. The method of claim 1, wherein the flavorant comprises D-limonene, methyl salicylate, vanillin, ethyl vanillin, benzaldehyde, methyl anthranilate, D,L-menthol, L-menthol and/or L-carvone.

3. The method of claim 1, wherein the tobacco product is a smoking article.

4. The method of claim 1, wherein the tobacco product is a smokeless tobacco product.

5. The method of claim 1, wherein the tobacco product is a dissolvable tobacco product.

6. The method of claim 1, wherein the tobacco product is an electronic cigarette.

7. The method of claim 5, wherein the tobacco product is a gum.

8. The method of claim 5, wherein the tobacco product is a lozenge.

9. The method of claim 5, wherein the tobacco product is snus.

10. The method of claim 5, wherein the tobacco product is a spray.

11. The method of claim 5, wherein the tobacco product is an orb or a stick.

12. The method of claim 1, wherein the tobacco product is a nicotine inhaler.

## Patentansprüche

1. Verfahren zum Überwachen der Verwendung eines Tabakprodukts durch ein Subjekt, wobei das Verfahren das Bereitstellen des Tabakprodukts an das Subjekt und, zu einem oder mehreren Zeitpunkten nach dem Bereitstellen des Tabakprodukts, Entnehmen einer Probe von ausgeatmetem Atem von dem Subjekt und Unterwerfen der entnommenen Probe einer Analyse zum Bestimmen der Menge und/oder Konzentration eines Markers umfasst, wobei die Menge und/oder Konzentration des Markers mit der Verwendung des Tabakproduktes durch das Subjekt in Beziehung steht, dadurch Überwachen der Verwendung des Tabakprodukts durch das Subjekt, **dadurch gekennzeichnet, dass**:
das Tabakprodukt einen Geschmacksstoff umfasst;
der Marker von dem Geschmacksstoff abgeleitet ist; und
der Marker 2-Propanol, 2-Butanol, 2-Pentanol, 3-Pentanol, 3-Methyl-2-butanol, 3-Hexanol, 2-Hexanol, 3-Methyl-2-pentanol, 4-Methyl-2-pentanol 2,4-Dimethyl-3-pentanol, 3-Methyl-3-hexanol, 2,6-Dimethyl-4-heptanol, 2-Heptanol, 3-Heptanol, 4-Heptanol, 5-Methyl-3-heptanol, 6-Methyl-3-heptanol, Cyclopentanol, Cyclohexanol, 4-lsopropylcyclohexanol und/oder Trimethylcyclohexanol umfasst.

2. Verfahren nach Anspruch 1, wobei der Geschmacksstoff D-Limonen, Methylsalicylat, Vanillin, Ethylvanillin, Benzaldehyd, Methylanthranilat, D,L-Menthol, L-Menthol und/oder L-Carvon umfasst.

3. Verfahren nach Anspruch 1, wobei das Tabakprodukt ein Rauchartikel ist.

4. Verfahren nach Anspruch 1, wobei das Tabakprodukt ein rauchfreies Tabakprodukt ist.

5. Verfahren nach Anspruch 1, wobei das Tabakprodukt ein auflösbares Tabakprodukt ist.

6. Verfahren nach Anspruch 1, wobei das Tabakprodukt eine elektronische Zigarette ist.

7. Verfahren nach Anspruch 5, wobei das Tabakprodukt ein Kaugummi ist.

8. Verfahren nach Anspruch 5, wobei das Tabakprodukt eine Lutschtablette ist.

9. Verfahren nach Anspruch 5, wobei das Tabakprodukt Kautabak ist.

10. Verfahren nach Anspruch 5, wobei das Tabakprodukt ein Spray ist.

11. Verfahren nach Anspruch 5, wobei das Tabakprodukt eine Kugel oder ein Stab ist.

12. Verfahren nach Anspruch 1, wobei das Tabakprodukt ein Nikotin-Inhalator ist.

## Revendications

1. Procédé de surveillance de l'utilisation d'un produit de tabac par un sujet, le procédé comprenant la fourniture du produit de tabac au sujet et, une ou plusieurs fois après la fourniture du produit de tabac, le prélèvement d'un échantillon d'expiration du sujet et la soumission de l'échantillon prélevé à une analyse pour déterminer une quantité et/ou une concentration d'un marqueur, dans lequel la quantité et/ou la concentration du marqueur est ou sont en rapport avec l'utilisation du produit de tabac par le sujet, en surveillant de la sorte l'utilisation du produit de tabac par le sujet, **caractérisé en ce que** :
le produit de tabac comprend un aromatisant ;
le marqueur est tiré de l'aromatisant ; et
le marqueur comprend du 2-propanol, du 2-butanol, du 2-pentanol, du 3-pentanol, du 3-méthyl-2-butanol, du 3-hexanol, du 2-hexanol, du 3-méthyl-2-pentanol, du 4-méthyl-2-pentanol, du 2,4-diméthyl-3-pentanol, du 3-méthyl-3-hexanol, du 2,6-diméthyl-4-heptanol, du 2-heptanol, du 3-heptanol, du 4-heptanol, du 5-méthyl-3-heptanol, du 6-méthyl-3-heptanol, du cyclopentanol, du cyclohexanol, du 4-isopropoxycyclohexanol et/ou du triméthylcyclohexanol.

2. Procédé selon la revendication 1, dans lequel l'aromatisant comprend du D-limonène, du salicylate de méthyle, de la vanilline, de l'éthylvanilline, du benzaldéhyde, de l'anthranilate de méthyle, du D,L-menthol, du L-menthol et/ou du L-carvone.

3. Procédé selon la revendication 1, dans lequel le produit de tabac est un article à fumer.

4. Procédé selon la revendication 1, dans lequel le produit de tabac est un produit de tabac sans fumée.

5. Procédé selon la revendication 1, dans lequel le produit de tabac est un produit de tabac dissoluble.

6. Procédé selon la revendication 1, dans lequel le produit de tabac est une cigarette électronique.

7. Procédé selon la revendication 5, dans lequel le produit de tabac est une gomme.

8. Procédé selon la revendication 5, dans lequel le produit de tabac est une tablette.

9. Procédé selon la revendication 5, dans lequel le produit de tabac est du tabac à chiquer (snus).

10. Procédé selon la revendication 5, dans lequel le produit de tabac est un spray.

11. Procédé selon la revendication 5, dans lequel le produit de tabac est une sphère ou un bâtonnet.

12. Procédé selon la revendication 1, dans lequel le produit de tabac est un inhalateur de nicotine.
